**Europäisches Patentamt**

**European Patent Office**

**Office européen des brevets**

(11) Veröffentlichungsnummer: **0 030 364**
**B1**

(12) **EUROPÄISCHE PATENTSCHRIFT**

(45) Veröffentlichungstag der Patentschrift:
20.03.85

(21) Anmeldenummer: **80107566.4**

(22) Anmeldetag: **03.12.80**

(51) Int. Cl.⁴: **A 61 B 6/14**

(54) **Zahnärztliche Röntgendiagnostikeinrichtung.**

(30) Priorität: **06.12.79 DE 2949199**

(43) Veröffentlichungstag der Anmeldung:
**17.06.81 Patentblatt 81/24**

(45) Bekanntmachung des Hinweises auf die Patenterteilung:
**20.03.85 Patentblatt 85/12**

(84) Benannte Vertragsstaaten:
**FR GB IT**

(56) Entgegenhaltungen:
**DE - A - 2 212 325**
**DE - U - 7 820 937**
**FR - A - 1 566 325**
**US - A - 1 920 684**
**US - A - 4 104 531**

**ORAL SURGERY, ORAL MEDICINE AND ORAL PATHOLOGY, Band 14, Nr. 10, 1961, Seiten 1178-1189 Mosby Company St. Louis, U.S.A. S. BLACKMAN: "Oral Roentgenology"**

(73) Patentinhaber: **Siemens Aktiengesellschaft, Berlin und München Wittelsbacherplatz 2, D-8000 München 2 (DE)**

(72) Erfinder: **Müther, Manfred, Adolf-Kolping-Strasse 20, D-6140 Bensheim (DE)**
Erfinder: **Behne, Ernst-August, Friedrich-Ebert-Strasse 29, D-6140 Bensheim (DE)**

## Beschreibung

Die Erfindung bezieht sich auf eine zahnärztliche Röntgendiagnostikeinrichtung, enthaltend einen Röntgenapparat mit Röntgengenerator, Röntgenröhre und intraoral eines Patientenmundes applizierbaren Applikator sowie ein auf den Applikator aufschiebbares Aufbissteil, welches eine den Applikator umgebende Führungshülse sowie eine daran befestigte Aufbissgabel enthält, welche eine die Einführtiefe in den Patientenmund festlegende Vertiefung oder Erhöhung enthält, an der bei appliziertem Aufbissteil zumindest die Frontzähne fixiert sind.

Bei bekannten zahnärztlichen Röntgendiagnostikeinrichtungen dieser Art (US-A-3 655 967) bildet der Röntgenapparat eine relativ voluminöse, am Fussboden aufstellbare Einheit, die im wesentlichen aus einem säulenartigen Kasten, in dem der Röntgengenerator und die Halterung für die Röntgenröhre angeordnet sind und einem mit dem Kasten fest verbundenen Stuhl für den Patienten besteht. Der fest mit der Einheit verbundene Stuhl enthält eine Kopfstütze, an der der Patientenkopf fixiert wird.

Zur Vorbereitung einer Aufnahme wird, nachdem die Winkelstellung zwischen der aufzunehmenden Zahnreihe und dem Röntgenapplikator eingestellt ist, dem Patienten vom Bedienungspersonal der Röntgenapplikator in die Mundhöhle eingeführt. Dabei gilt zu beachten, dass der Applikator auf der Symmetrieachse des aufzunehmenden Kieferbogens angeordnet ist. Das Bedienungspersonal hat bei dieser Anordnung und Handhabung keine Einsicht in die Mundhöhle des Patienten, so dass die Gefahr besteht, dass Teile der Mundhöhle vom Röntgenapplikator beim Einführen berührt werden, wodurch es beim Patienten sehr leicht zu einem Brechreiz kommen kann. Psychologisch ungünstig ist ausserdem, dass der Patient keine Möglichkeit einer Einflussnahme auf den Ablauf des Applikatoreinführens hat.

Dies scheint auch bei einer anderen bekannten Einrichtung (FR-A-1 566 325) der Fall zu sein, wo man zwar ein auf den Applikator aufsetzbares Aufbissteil vorgesehen hat, dieses Teil aber in fester, verdrehsicherer Zuordnung zum Applikator angeordnet hat. Dieses Aufbissteil dient ausserdem dazu, den Röntgenfilm zu fixieren, wodurch sich zwangsläufig ergibt, dass das Aufbissteil zunächst auf den Applikator aufgesetzt und dort verdrehsicher fixiert werden muss, bevor Applikator und Aufbissteil in den Patientenmund eingeführt werden können.

Aus US-A-4 104 531 schliesslich ist eine zahnärztliche Röntgendiagnostikeinrichtung bekannt, bei der auf den in den Patientenmund einführbaren Applikator ein Zungenhalter aufgesetzt werden kann. Dieser Zungenhalter dient einerseits dazu, die Zunge aus dem gewünschten Strahlungsbereich herauszuhalten, andererseits soll er ein Schutzschild sein, um dahinterliegende Teile von unerwünschter Strahlung zu schützen. Dieser Zungenhalter ist kein Aufbissteil im vorgenannten Sinne. Auch bei dieser bekannten Einrichtung ist ein leichtes Einführen des vorher vom Patienten aufgenommenen Aufbissteils nicht möglich.

Aufgabe der Erfindung ist es, eine gegenüber dem Stand der Technik verbesserte zahnärztliche Röntgendiagnostikeinrichtung anzugeben, insbesondere mit dem Ziel, das Einführen und Ausrichten des Applikators in den Patientenmund sowohl für die Bedienungsperson als auch für den Patienten leichter und angenehmer durchführen und die Einrichtung baulich kleiner gestalten zu können.

Die gestellte Aufgabe wird bei einer zahnärztlichen Röntgendiagnostikeinrichtung der eingangs genannten Gattung erfindungsgemäss dadurch gelöst, dass das vom Applikator abziehbare, gesonderte Aufbissteil unabhängig vom Applikator im Patientenmund applizierbar ist, die Führungshülse an dem dem Patientenmund zugewandten Ende stirnseitig geschlossen und im Innendurchmesser grösser bemessen ist als der Aussendurchmesser des Applikators, dass das Aufbissteil mit geringem Spiel auf den Applikator aufgesetzt werden kann, und dass weiterhin der Röntgenapparat als auf einen Tisch stellbare oder an einer Wand befestigbare Einheit ausgebildet ist, welche ein ortsfestes Tragteil enthält, an dem mittels einer horizontalen Achslagerung ein Schwenkteil mit der Röntgenröhre und dem Applikator schwenkbar gehaltert ist.

Vorteilhafte Ausgestaltungen und Weiterbildungen der Erfindung sind in den Unteransprüchen enthalten.

Durch die Verwendung eines Aufbissteiles, das vorher im Mund des Patienten von diesem selbst appliziert werden kann, ist es möglich, auf einen speziellen Stuhl sowie auf zusätzliche Halteelemente für den Kopf des Patienten zu verzichten. Der eigentliche Röntgenapparat ist vorteilhafterweise platzsparend als Wand- oder Tischgerät ausgebildet, wodurch die bei bekannten Einrichtungen vorhandenen, mehrgliedrigen aufwendigen Tragarmkonstruktionen, die das exakte Einstellen des Applikators auf den im Stuhl fixierten Patienten ermöglichen, entfallen können. Der Patient kann also bei der erfindungsgemässen Einrichtung auf einem beliebigen Stuhl vor dem Röntgenapparat Platz nehmen. Das Schwenklager lässt eine Veränderung der Winkeleinstellung der Röntgenröhre mit Applikator und damit eine gewisse Höheneinstellbarkeit zu. Zur Vorbereitung einer Aufnahme appliziert sich der Patient den Aufbissteil selbst in den Mund; durch Annäherung an den Applikator kann er sich sodann den Röntgenapplikator applizieren. Da der Röntgenapplikator allseitig von der Führungshülse des Aufbissteils umgeben ist, kann es zu keiner mechanischen Reizung der Mundhöhle kommen.

Zwei Ausführungsbeispiele der Erfindung werden nachfolgend anhand der Zeichnung näher erläutert. Es zeigen:

Fig. 1 ein erstes Ausführungsbeispiel einer zahnärztlichen Röntgendiagnostikeinrichtung nach der Erfindung in einer schaubildlichen Darstellung,

Fig. 2 ein weiteres Ausführungsbeispiel in schaubildlicher Darstellung.

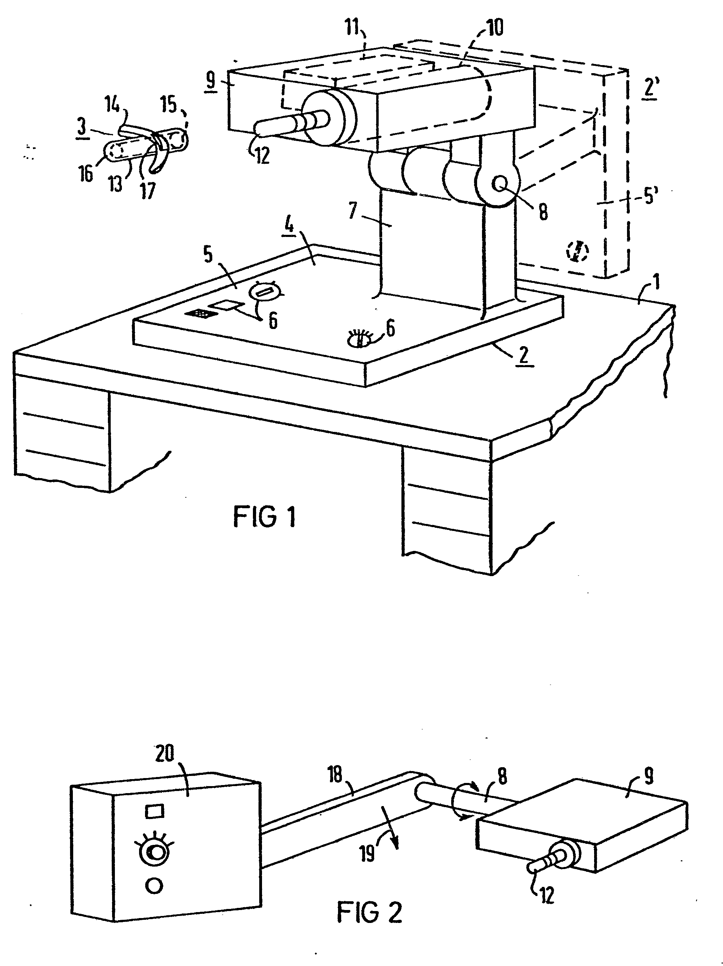

Die Fig. 1 zeigt eine zahnärztliche Röntgendiagnostikeinrichtung mit einem auf einem Tisch 1 plazierten Röntgenapparat 2 und einem dem Röntgenapparat zugeordneten Aufbissteil 3, der, wie nachfolgend noch näher erläutert wird, vom Patienten appliziert wird und dazu dient, das Einführen des Röntgenapplikators in den Patientenmund zu erleichtern.

Der Röntgenapparat 2 enthält ein Tragteil 4 mit einem flachen, rechteckigen ersten Gehäuse 5, in dem mehrere Einstell- und Kontrollorgane 6 angeordnet sind. Am hinteren Ende des Gehäuses 5 erstreckt sich vertikal eine Tragsäule 7, an deren freiem Ende sich ein Schwenklager 8 befindet. Mittels des Schwenklagers 8 ist ein zweites Gehäuse 9 schwenkbar gehalten, welches eine Röntgenröhre 10, einen mit 11 schematisch angedeuteten Röntgengenerator bzw. wesentliche Teile von diesem (z. B. den Hochspannungstrafo) sowie einen in den Patientenmund einführbaren Applikator 12 aufnimmt.

Das Schwenklager 8 ist so ausgebildet, dass die beiden Gehäuse 5 und 9 um wenigstens 90° relativ zueinander verstellt werden können, so dass unter Beibehaltung der Lage des Gehäuses 9, wie dargestellt, das Gehäuse 5 in die gestrichelt eingezeichnete Position, die einer Wandausführung entspricht, gebracht werden kann. Um den gleichen Röntgenapparat alternativ als Tisch- oder Wandgerät benutzen zu können, ist das Gehäuse 5 bereits für eine Wandbefestigung vorbereitet.

Das Aufbissteil 3 enthält eine Führungshülse 13 und eine auf dieser angeordneten Aufbissgabel 14. Die Führungshülse 13, die ebenso wie die Aufbissgabel 13 vorteilhafterweise aus sterilisierbarem Material gefertigt ist, hat eine Länge, die etwa der Länge des Applikators 12 entspricht und eine Öffnung 15, die geringfügig grösser ist als der Aussendurchmesser des Applikators 12. Dadurch kann dieser mit geringem Spiel in die Hülse 13 eingeführt werden. Das dem Patientenmund zugewandte Ende der Hülse 13 ist verschlossen und bildet im aufgesetzten Zustand einen Anschlag 16 für den Applikator 12. Die Aufbissgabel 14 ist der durchschnittlichen Kieferform angepasst und mit keilförmigen Enden versehen. Sie weist ferner eine Abstufung 17 in Form einer Erhebung oder Einbuchtung auf, die bei appliziertem Aufbissteil eine Anlage und Fixierung für die Frontzähne gibt. Der Aufbissteil kann so vom Patienten exakt positioniert und fixiert werden. Eine detailliertere Beschreibung des Aufbissteils mit dem Hinweis auf mögliche Varianten ist in dem deutschen Gebrauchsmuster entsprechend dem Aktenzeichen G 7 820 937.7 enthalten.

Die Kombination von Aufbissteil und Tisch- bzw. Wandgerät lässt es zu, dass der Patient auf einem beliebigen Stuhl vor dem Röntgengerät Platz nehmen kann. Das Schwenklager 8 ermöglicht eine Veränderung der Winkeleinstellung des Applikators 12 und damit des Röntgenstrahles. Durch die Anordnung des Schwenklagers 8 am hinteren unteren Ende des Gehäuses 9 ergibt sich eine gewisse Höheneinstellbarkeit. Zur Vorbereitung einer Röntgenaufnahme wird der Aufbissteil 3 vom Patienten zunächst im Mund appliziert. Danach appliziert er sich den Applikator 12, indem er durch Annäherung an den Röntgenapparat und Applikator 12 diesen in die Öffnung der Führungshülse 13 einführt. Eine Reizung der Mundhöhle durch den Applikator ist ausgeschlossen, da dieser allseitig von der Führungshülse 13 umschlossen ist.

Die Fig. 2 zeigt eine andere Ausführungsform eines an einer Wand befestigbaren Röntgenapparates nach der Erfindung. Bei diesem Ausführungsbeispiel ist das Gehäuse 9 mit dem Applikator 12 auf einem Parallelogrammtragarm 18, der in Richtung des Pfeiles 19 bewegbar ist, schwenkbar gehalten. Das Schwenklager 8 ist auch hier wiederum am hinteren unteren Ende des Gehäuses 9 gehalten. Ein Wandgehäuse 20 nimmt den Parallelogrammarm 18 auf, und zwar so, dass dieser etwa parallel zur Wand verläuft. Für die Bewegung des Parallelogrammarmes enthält das Gehäuse 20 seitlich eine entsprechende Aussparung. Die Zwischenschaltung dieses eingliedrigen Parallelogrammarmes hat den Vorzug, dass bei unterschiedlichen Körpergrössen der Patienten der einmal eingestellte Strahlerwinkel gleich bleibt. Eine Schwenkung des Strahlers um das Schwenklager 8 ermöglicht auch Aufnahmen am liegenden Patienten.

Ein wesentlicher Vorteil der erfindungsgemässen Einrichtung ist, dass der Patient die Hilfsvorrichtung in Form des Aufbissteils 3 zum Einführen des Applikators selbst in den Mund nehmen kann und er sodann ohne fremde Hilfe den Applikator, ohne einen Brechreiz auszulösen, in die Öffnung der Führungshülse einführen kann. Ein zu tiefes oder anderweitig nicht sachgemässes Einführen des Applikators ist ausgeschlossen. Ein weiterer wesentlicher Vorzug ist darin zu sehen, dass durch den Aufbissteil eine exakte Positionierung an den Applikator ermöglicht wird, wodurch die bisher notwendigen Positionierungsmassnahmen (mit dem Röntgenapparat verbundener Stuhl mit Kopfhalterung) entbehrlich werden. Der Röntgenapparat selbst kann so vorteilhafterweise als platzsparendes Tisch- oder Wandgerät ausgebildet sein.

**Patentansprüche**

1. Zahnärztliche Röntgendiagnostikeinrichtung, enthaltend einen Röntgenapparat (2) mit Röntgengenerator (11), Röntgenröhre (10) und intraoral eines Patientenmundes applizierbaren Applikator (12) sowie ein auf den Applikator (12) aufschiebbares Aufbissteil (3), welches eine den Applikator umgebende Führungshülse (13) sowie eine daran befestigte Aufbissgabel (14) enthält, welche eine die Einführtiefe in den Patientenmund festlegende Vertiefung oder Erhöhung (17) enthält, an der bei appliziertem Aufbissteil zumindest die Frontzähne fixiert sind, dadurch gekennzeichnet, dass das vom Applikator abziehbare, gesonderte Aufbissteil unabhängig vom Applikator im Patientenmund applizierbar ist, die Führungshülse (13) an dem dem Patientenmund zugewandten Ende stirnseitig geschlossen und im Innendurchmesser grösser bemessen ist als der Aussendurchmesser des Applikators (12), dass das Aufbissteil mit geringem Spiel auf den Applikator aufgesetzt werden kann, und dass weiterhin der Röntgenapparat (2) als auf einen Tisch stellbare oder an einer Wand befestigbare Einheit ausgebildet ist, welche ein ortsfestes Tragteil (4, 20) enthält, an dem mittels einer horizontalen Achslagerung (8) ein Schwenkteil (9) mit der Röntgenröhre (10) und dem Applikator (12) schwenkbar gehalten ist.

2. Zahnärztliche Röntgendiagnostikeinrichtung nach Anspruch 1, dadurch gekennzeichnet, dass in dem Schwenkteil (9) auch wenigstens der Hochspannungsteil des Röntgengenerators (11) angeordnet ist.

3. Zahnärztliche Röntgendiagnostikeinrichtung nach Anspruch 2, dadurch gekennzeichnet, dass die Achslagerung (8) für das Schwenkteil (9) am unteren hinteren Ende desselben angeordnet ist.

4. Zahnärztliche Röntgendiagnostikeinrichtung nach einem der Ansprüche 1 bis 3, dadurch gekennzeichnet, dass das Tragteil (4) ein flaches Standgehäuse (5), in dem diverse Einstell- und Bedienungsorgane (6) angeordnet sind, enthält, und dass an dem Standgehäuse (5), vorzugsweise von dessen hinterem Ende aus, eine sich im wesentlichen vertikal erstreckende Tragsäule (7) zur Aufnahme der Achslagerung (8) angeordnet ist.

5. Zahnärztliche Röntgendiagnostikeinrichtung nach einem der Ansprüche 1 bis 4, dadurch gekennzeichnet, dass das Standgehäuse (5) zur wahlweisen Aufstellung bzw. Befestigung auf einem Tisch (1) oder an einer Wand ausgebildet ist.

6. Zahnärztliche Röntgendiagnostikeinrichtung nach einem der Ansprüche 1 bis 3, dadurch gekennzeichnet, dass das Tragteil (4, 20) zur Befestigung an einer Wand ausgebildet ist und die Schwenklagerung (8) am freien Ende eines am Tragteil (4, 20), vorzugsweise seitlich und im wesentlichen parallel zur Wand angeordneten Parallelogrammtragarm (18) angeordnet ist.

## Claims

1. A dental X-ray diagnostic device comprising an X-ray apparatus (2) having an X-ray generator (11), an X-ray tube (10) and an applicator (12), which can be applied intraorally of a patient's mouth, as well as a piece (3) for biting-on which can be slid on to the applicator (12) and which comprises a guide sleeve (13) which surrounds the applicator, and a fork (14) for biting-on which is secured thereto and which has a recess or raised part (17) which determines the depth of insertion into the patient's mouth and on which at least the front teeth are fixed when the piece for biting-on is applied, characterised in that the separate piece (3) for biting-on, which can be removed from the applicator (12), can be applied in the patient's mouth independently of the applicator; at the end facing the patient's mouth, the guide sleeve (13) is closed at the end face thereof and is so dimensioned as to have an internal diameter larger than the outer diameter of the applicator (12); that the piece (3) for biting-on can be placed on to the applicator with little play; and that, moreover, the X-ray apparatus (2) is designed as a unit which can be placed on a table or secured to a wall and which comprises a stationary support member (4, 20), on which a swivelling member (9) with the X-ray tube (10) and the applicator, is pivotably mounted by means of a horizontal axle bearing (8).

2. A dental X-ray diagnostic device as claimed in Claim 1, characterised in that at least the high-voltage part of the X-ray generator (11) is arranged in the swivelling member (9).

3. A dental X-ray diagnostic device as claimed in Claim 2, characterised in that the axle bearing (8) for the swivelling member (9) is arranged at the lower rear end thereof.

4. A dental X-ray diagnostic device as claimed in one of Claims 1 to 3, characterised in that the support member (4) comprises a flat stationary housing (5) in which various adjusting and operating elements (6) are arranged; and that on the stationary housing (5), preferably at the rear end thereof, there is arranged a supporting column (7) which extends essentially vertically and which serves to accommodate the axle bearing (8).

5. A dental X-ray diagnostic device as claimed in one of Claims 1 to 4, characterised in that the stationary housing (5) is designed for selective erection or mounting, as the case may be, on a table (1), or on a wall.

6. A dental X-ray diagnostic device as claimed in one of Claims 1 to 3, characterised in that the support member (4, 20) is designed to be mounted on a wall and the pivot bearing (8) is arranged at the free end of a supporting parallelogram arm (18) which is preferably arranged at the support member (4, 20) so as to be laterally and essentially parallel to the wall.

## Revendications

1. Appareil de radiodiagnostic dentaire, contenant un groupe radiogène comportant un générateur radiologique (11), un tube à rayons X (10) et un applicateur (12) pouvant être appliqué par voie orale dans la bouche du patient, ainsi qu'un embout à mordre (3) pouvant être emmanché sur l'applicateur (12) et qui contient une douille de guidage (13) entourant l'applicateur une pièce fourchue à mordre (14) fixée sur cette douille et qui comporte un renfoncement ou un bombement (17), qui détermine la profondeur de pénétration dans la bouche du patient et contre lequel au moins les dents avant sont maintenues lorsque l'embout à mordre est appliqué, caractérisé par le fait que l'embout à mordre indépendant (3), qui peut être retiré de l'applicateur (12), peut être appliqué dans la bouche du patient d'une manière indépendante de l'applicateur, que la douille de guidage (13) est fermée sur sa face frontale au niveau de l'extrémité tournée vers la bouche du patient et possède un diamètre intérieur supérieur au diamètre extérieur de l'applicateur (12), que l'embout à mordre (3) peut être monté avec un faible jeu sur l'applicateur et qu'en outre le groupe radiogène (2) est réalisé sous la forme d'une unité pouvant être posée sur une table ou fixée à une paroi et qui comporte un organe de support fixe (4, 20) sur lequel une partie pivotante (9) portant le tube à rayons X (10) et l'applicateur (12) est maintenue de façon à pouvoir basculer au moyen d'un axe horizontal de tourillonnage (8).

2. Appareil de radiodiagnostic dentaire suivant la revendication 1, caractérisé par le fait qu'au moins la partie à haute tension du générateur radiologique (11) est également disposée dans la partie pivotante (9).

3. Appareil de radiodiagnostic dentaire selon la revendication 2, caractérisé par le fait que l'axe de tourillonnage (8) de la partie pivotante (9) est monté sur l'extrémité arrière inférieure de cette partie.

4. Appareil de radiodiagnostic dentaire suivant l'une des revendications 1 à 3, caractérisé par le fait que l'organe de support (4) contient un boîtier de base plat (5), dans lequel se trouvent disposés différents organes de réglage et de commande (6), et qu'un pied de support (7), qui s'étend essentiellement verticalement et qui est destiné à supporter l'axe de tourillonnage (8), est disposé sur le boîtier de base (5), de préférence au niveau de l'extrémité arrière de ce boîtier.

5. Appareil de radiodiagnostic suivant l'une des revendications 1 à 4, caractérisé par le fait que le boîtier (5) est réalisé de manière à pouvoir être au choix apposé ou fixé sur une talbe (1) ou à un mur.

6. Appareil de radiodiagnostic dentaire suivant l'une des revendications 1 à 3, caractérisé par le fait que l'organe de support (4, 20) est réalisé de manière à pouvoir être fixé à un mur et que l'axe de tourillonnage (8) est disposé sur l'extrémité libre d'un bras de support (18) en forme de parallèlogramme qui est monté sur l'organe de support (4, 20) en étant disposé de préférence latéralement et essentiellement parallèlement au mur.

FIG 1

FIG 2